(19) Europäisches Patentamt
European Patent Office
Office européen des brevets

(11) EP 4 082 544 A1

(12) EUROPEAN PATENT APPLICATION
published in accordance with Art. 153(4) EPC

(43) Date of publication:
**02.11.2022 Bulletin 2022/44**

(21) Application number: **20905210.9**

(22) Date of filing: **25.12.2020**

(51) International Patent Classification (IPC):
***A61K 31/436*** *(2006.01)* ***A61K 9/10*** *(2006.01)*
***A61K 47/26*** *(2006.01)* ***A61K 47/38*** *(2006.01)*
***A61P 27/02*** *(2006.01)*

(52) Cooperative Patent Classification (CPC):
**A61K 9/10; A61K 31/436; A61K 47/26; A61K 47/38; A61P 27/02**

(86) International application number:
**PCT/JP2020/048738**

(87) International publication number:
**WO 2021/132565 (01.07.2021 Gazette 2021/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.12.2019 JP 2019237226**

(71) Applicant: **Santen Pharmaceutical Co., Ltd.**
**Kita-ku**
**Osaka-shi**
**Osaka 530-8552 (JP)**

(72) Inventors:
- **FUJISAWA Toyomi**
**Ikoma-shi, Nara 630-0101 (JP)**
- **AKUTAGAWA Ayaka**
**Osaka-shi, Osaka 530-8552 (JP)**

(74) Representative: **Müller-Boré & Partner Patentanwälte PartG mbB**
**Friedenheimer Brücke 21**
**80639 München (DE)**

(54) **AQUEOUS SUSPENSION COMPOSITION CONTAINING SIROLIMUS OR SALT THEREOF**

(57) The purpose of the present invention is to provide an aqueous suspension composition comprising poorly water-soluble sirolimus or a salt thereof for ophthalmology, in particular, used for topical administration, such as a less invasive eye drop.

The present invention is an ophthalmic aqueous suspension composition comprising sirolimus or a salt thereof and a surfactant, wherein the aqueous suspension composition has a pH of 4 to 6.



Processed by Luminess, 75001 PARIS (FR)

**Description**

TECHNICAL FIELD

The present invention relates to an aqueous suspension composition comprising sirolimus or a salt thereof (hereinafter, they are sometimes simply and generally referred to as "sirolimus"). In addition, the present invention also relates to a method of suppressing decomposition and/or agglomeration of sirolimus in an aqueous suspension composition comprising sirolimus or a salt thereof.

BACKGROUND ART

Sirolimus (also called rapamycin) is a macrolide compound discovered from actinomycete metabolites, and has an immunosuppressive action. Thus, its oral preparation for the prophylaxis of rejection upon organ transplantation or for the treatment of lymphangioleiomyomatosis has been approved as a drug. In addition, sirolimus has been known to be useful for the treatment of autoimmune diseases, inflammatory diseases, fungal infections, leukemia/lymphoma, hyperproliferative vascular disease or the like.

In the field of ophthalmology, for example, Patent Document 1 describes a method of treating ocular inflammation in a mammal that requires the treatment including administering to the mammal an effective anti-inflammatory amount of rapamycin. Patent Document 2 describes an ophthalmic composition containing an mTOR inhibitor such as sirolimus, everolimus, or temsirolimus, a first surfactant with an HLB value greater than about 10, and a second surfactant with an HLB value greater than about 13. Patent Document 3 describes a prophylactic and/or therapeutic agent for meibomian dysfunction, comprising a compound such as sirolimus or deforolimus or a pharmaceutically acceptable salt thereof as an active ingredient. Meanwhile, sirolimus is almost insoluble in water because its chemical structure does not contain any functional groups that ionize in a weakly acidic, neutral, or weakly basic pH range.

In addition, the General Rules for Preparations of the Japanese Pharmacopoeia, 17th Edition, states that particles in an ophthalmic suspension should typically have a maximum particle size of 75 μm or less.

PRIOR ART DOCUMENTS

Patent Documents

Patent Document 1: JP H5-194212 A
Patent Document 2: JP 2010-540682 A
Patent Document 3: WO2014/142146A1

SUMMARY OF INVENTION

Technical Problem

It is an interesting problem to provide an aqueous suspension composition containing poorly water-soluble sirolimus for ophthalmology, in particular, used for topical administration, such as a less invasive eye drop.

Solution to Problem

The present inventors have found that although a solution-type aqueous composition can be prepared by adding a solubilizing agent to poorly water-soluble sirolimus, sirolimus in the composition is likely to decompose; on the other hand, in an aqueous composition in which sirolimus is simply suspended in water, the suspension is likely to agglomerate. The present inventors have further conducted intensive research on sirolimus-containing ophthalmic aqueous compositions. As a result, it has been found that the pH of sirolimus-containing aqueous composition, the average particle size of sirolimus, the content of additive(s), and the like affect the decomposition and agglomeration of sirolimus; and in the sirolimus-containing aqueous suspension composition described in detail later, sirolimus decomposition and/or agglomeration of sirolimus can be suppressed to a minimum. In this way, the present invention has been completed.

Specifically, the present invention provides the following.

(1) An ophthalmic aqueous suspension composition comprising sirolimus or a salt thereof and a surfactant, wherein the aqueous suspension composition has a pH of 4 to 6.

(2) The aqueous suspension composition according to (1), wherein a content ratio of the surfactant to sirolimus or a salt thereof is more than 0.01 parts by weight based on 1 part by weight of a content of sirolimus or a salt thereof, and sirolimus or a salt thereof in the aqueous suspension composition has an average particle size of 45 μm or less.
(3) The aqueous suspension composition according to (1), wherein a content ratio of the surfactant to sirolimus or a salt thereof is more than 0.01 parts by weight based on 1 part by weight of a content of sirolimus or a salt thereof, and sirolimus or a salt thereof in the aqueous suspension composition has an average particle size of 15 μm or less.
(4) The aqueous suspension composition according to (1), wherein a content ratio of the surfactant to sirolimus or a salt thereof is more than 0.01 parts by weight based on 1 part by weight of a content of sirolimus or a salt thereof, and sirolimus or a salt thereof in the aqueous suspension composition has an average particle size of 10 μm or less.
(5) The aqueous suspension composition according to any one of (1) to (4), wherein the aqueous suspension composition has a pH of 5.
(6) The aqueous suspension composition according to any one of (1) to (5), wherein a content of sirolimus or a salt thereof is from 0.01 to 1% (w/v).
(7) The aqueous suspension composition according to any one of (1) to (6), wherein sirolimus or a salt thereof has an average particle size of 2.5 μm or less.
(8) The aqueous suspension composition according to any one of (1) to (7), wherein the surfactant is one or more selected from the group consisting of polyoxyethylene fatty acid esters, polyoxyethylene sorbitan fatty acid esters, polyoxyethylene hydrogenated castor oils, polyoxyl castor oils, and polyoxyethylene alkyl ether phosphates.
(9) The aqueous suspension composition according to any one of (1) to (7), wherein the surfactant is one or more selected from the group consisting of polyoxyl 40 stearate, polysorbate 80, polyoxyl 35 castor oil, and sodium polyoxyethylene cetyl ether phosphate.
(10) The aqueous suspension composition according to any one of (1) to (7), wherein the surfactant is polysorbate 80.
(11) The aqueous suspension composition according to any one of (1) to (10), comprising a dispersant.
(12) The aqueous suspension composition according to (11), wherein the dispersant is one or more selected from the group consisting of cellulosic polymers, polyalcohols, polyvinylpyrrolidone, and mucopolysaccharides.
(13) The aqueous suspension composition according to any one of (1) to (12), further comprising one or more selected from the group consisting of buffering agents, tonicity agents, stabilizers, antioxidants, preservatives, and pH adjusters.
(14) The aqueous suspension composition according to (13), wherein the preservatives are one or more selected from the group consisting of invert soaps, parabens, sorbic acid or salts thereof, chlorobutanol, and silver nitrate.
(15) The aqueous suspension composition according to any one of (1) to (14), which is an eye drop.
(16) An ophthalmic aqueous suspension composition comprising sirolimus or a salt thereof and polysorbate 80, wherein

a content of sirolimus or a salt thereof is from 0.01 to 1% (w/v),
a content ratio of the polysorbate 80 to sirolimus or a salt thereof is from 0.1 to 10 parts by weight based on 1 part by weight of a content of sirolimus or a salt thereof,
sirolimus or a salt thereof in the aqueous suspension composition has an average particle size of 2.5 μm or less, and
the aqueous suspension composition has a pH of 4 to 6.

(17) A method of suppressing agglomeration of sirolimus or a salt thereof in an aqueous suspension composition comprising sirolimus or a salt thereof and a surfactant, comprising adjusting a pH of the aqueous suspension composition to 4 to 6 and setting an average particle size of sirolimus or a salt thereof in the aqueous suspension composition to 45 μm or less.
(18) A method of suppressing agglomeration of sirolimus or a salt thereof in an aqueous suspension composition comprising sirolimus or a salt thereof and a surfactant, comprising adjusting a pH of the aqueous suspension composition to 4 to 6 and setting an average particle size of sirolimus or a salt thereof in the aqueous suspension composition to 15 μm or less.
(19) A method of suppressing agglomeration of sirolimus or a salt thereof in an aqueous suspension composition comprising sirolimus or a salt thereof and a surfactant, comprising adjusting a pH of the aqueous suspension composition to 4 to 6 and setting an average particle size of sirolimus or a salt thereof in the aqueous suspension composition to 10 μm or less.
(20) A method of suppressing decomposition of sirolimus in an aqueous suspension composition comprising sirolimus or a salt thereof and a surfactant, comprising adjusting a pH of the aqueous suspension composition to 4 to 6.
(21) A method of suppressing agglomeration of sirolimus or a salt thereof in an aqueous suspension composition comprising sirolimus or a salt thereof and a surfactant, comprising adjusting a pH of the aqueous suspension composition to 4 to 6.

(22) A method of suppressing agglomeration of sirolimus or a salt thereof in an aqueous suspension composition comprising sirolimus or a salt thereof and a surfactant, comprising setting an average particle size of sirolimus or a salt thereof in the aqueous suspension composition to 45 μm or less.
(23) A method of suppressing agglomeration of sirolimus or a salt thereof in an aqueous suspension composition comprising sirolimus or a salt thereof and a surfactant, comprising setting an average particle size of sirolimus or a salt thereof in the aqueous suspension composition to 15 μm or less.
(24) A method of suppressing agglomeration of sirolimus or a salt thereof in an aqueous suspension composition comprising sirolimus or a salt thereof and a surfactant, comprising setting an average particle size of sirolimus or a salt thereof in the aqueous suspension composition to 10 μm or less.

Further, the present invention provides the following.

(25) A method of treating ocular disease, comprising administering to a patient in need of the treatment a therapeutically effective amount of the aqueous suspension composition according to any one of (1) to (16).
(26) The treatment method according to (25), wherein the ocular disease is anterior eye disease.
(27) Use of the aqueous suspension composition according to any one of (1) to (16) for the manufacture of a medicament for treating and/or preventing ocular disease.
(28) The use according to (27), wherein the ocular disease is anterior eye disease.
(29) The aqueous suspension composition according to any one of (1) to (16) for use in the treatment and/or prevention of ocular disease.
(30) The aqueous suspension composition according to (29), wherein the ocular disease is anterior eye disease.

Any two or more configurations of (1) to (30) described above can be selected and combined.

Advantageous Effect of Invention

The present invention can provide an aqueous suspension composition comprising poorly water-soluble sirolimus or a salt thereof for ophthalmology, in particular, used for topical administration, such as a less invasive eye drop.

DESCRIPTION OF EMBODIMENTS

Hereinafter, the present invention will be described in detail.
In the aqueous suspension composition of the present invention, "sirolimus" is also referred to as "rapamycin", and is a compound represented by the following formula:

[Chemical Formula 1]

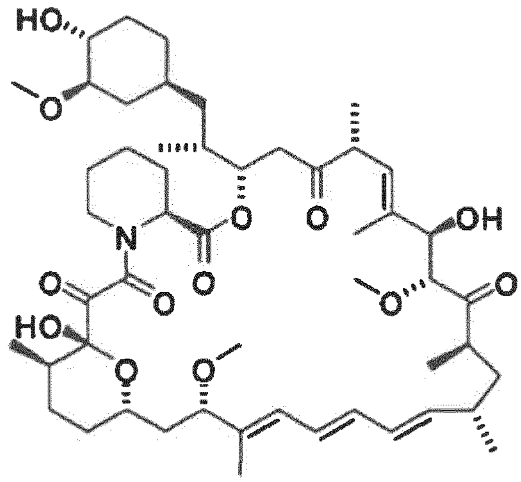

In the aqueous suspension composition of the present invention, contained sirolimus may be a racemate or enantiomer.
In the aqueous suspension composition of the present invention, contained sirolimus may be a salt and is not particularly limited as long as the salt is pharmaceutically acceptable. Sirolimus or a salt thereof may be produced in accordance with an ordinary method in the field of synthetic organic chemistry. Also, commercially available ones may

be used.

In the aqueous suspension composition of the present invention, examples of the pharmaceutically acceptable salt include: a salt with an inorganic acid such as hydrochloric acid, hydrobromic acid, hydroiodic acid, nitric acid, sulfuric acid, phosphoric acid; a salt with an organic acid such as acetic acid, fumaric acid, maleic acid, succinic acid, citric acid, tartaric acid, adipic acid, gluconic acid, glucoheptic acid, glucuronic acid, terephthalic acid, methanesulfonic acid, lactic acid, hippuric acid, 1,2-ethanedisulfonic acid, isethionic acid, lactobionic acid, oleic acid, pamoic acid, polygalacturonic acid, stearic acid, tannic acid, trifluoromethanesulfonic acid, benzenesulfonic acid, p-toluenesulfonic acid, lauryl sulfate ester, methyl sulfate, naphthalenesulfonic acid, sulfosalicylic acid; a quaternary ammonium salt with methyl bromide, methyl iodide, or the like; a salt with a halogen ion such as bromine ion, chlorine ion, iodine ion; a salt with an alkali metal such as sodium, potassium; and a salt with an alkaline earth metal such as magnesium, calcium. The sirolimus contained in the present aqueous suspension composition is preferably sirolimus in a free form (free sirolimus).

In the aqueous suspension composition of the present invention, sirolimus or a salt thereof may be in the form of hydrate or solvate.

In the aqueous suspension composition of the present invention, geometric or optical isomers of sirolimus or a salt thereof may be present. In this case, the isomers or their salts are also included in the scope of the present invention. In addition, proton tautomers of sirolimus or a salt thereof may be present. In this case, the tautomers or salts thereof are also included in the scope of the present invention.

In the aqueous suspension composition of the present invention, crystal polymorphs and a crystal polymorph group (crystal polymorph system) of sirolimus or a salt thereof (including hydrates or solvates) may be present. In this case, the crystal polymorphs and crystal polymorph group (crystal polymorph system) are also included in the scope of the present invention. The "crystal polymorph group" (crystal polymorph system) means individual crystal forms at each stage or during the entire process when the crystal forms change depending on the conditions and states of manufacture, crystallization, storage, or the like of the crystals (note that these conditions includes a formulation state).

In the aqueous suspension composition of the present invention, the upper limit of the content of sirolimus or a salt thereof (hereinafter, the "content" is also referred to as the "concentration") is 5% (w/v), and 2% (w/v) or less is preferable. In addition, the lower limit of the content of sirolimus or a salt thereof should be the amount that does not cause complete dissolution, and 0.001% (w/v) or more is preferable. For example, the content of sirolimus or a salt thereof is preferably from 0.001% to 5% (w/v), more preferably from 0.001% to 2% (w/v), still more preferably from 0.01% to 2% (w/v), further preferably from 0.01% to 1% (w/v), and particularly preferably from 0.01% to 0.1% (w/v). Specifically, the content of sirolimus or a salt thereof may be 0.01% (w/v), 0.02% (w/v), 0.025% (w/v), 0.03% (w/v), 0.04% (w/v), 0.05% (w/v), 0.06% (w/v), 0.07% (w/v), 0.075% (w/v), 0.08% (w/v), 0.09% (w/v), or 0.1% (w/v).

Note that in the present invention, "% (w/v)" means the mass (g) of a component of interest contained in 100 mL of an aqueous suspension composition of the present invention. In the present invention, in the case of containing a salt of sirolimus, the value is the content of the salt of sirolimus. In addition, when sirolimus or a salt thereof is added in the form of a hydrate or solvate in the present invention, the value is the content of a hydrate or solvate of sirolimus or a salt thereof. Hereinafter, the same applies unless otherwise indicated.

In the aqueous suspension composition of the present invention, if the average particle size of sirolimus or a salt thereof is large, agglomeration is likely to occur in the aqueous suspension composition. Thus, the smaller the average particle size of sirolimus or a salt thereof in the aqueous suspension composition, the better. The average particle size is preferably 45 μm or less, more preferably 15 μm or less, and still more preferably 10 μm or less. The average particle size of sirolimus or a salt thereof may be 9 μm or less, 8 μm or less, 7 μm or less, 6.4 μm or less, 6 μm or less, 5 μm or less, 4 μm or less, or 3 μm or less, more preferably 2.5 μm or less or less than 2.5 μm, still more preferably 2 μm or less, 1.5 μm or less, or 1 μm or less, further preferably 0.5 μm or less, and particularly preferably 0.3 μm or less. The lower limit of the average particle size is not particularly limited as long as the particles with the average particle size can be produced. The average particle size is, for example, more than 0 μm or 0.001 μm or more. The average particle size of sirolimus or a salt thereof may be from 0.001 to 45 μm, preferably from 0.001 to 15 μm or from 0.001 to 10 μm, more preferably from 0.001 to 8 μm, still more preferably from 0.001 to 5 μm, further preferably from 0.001 to 2.5 μm, particularly preferably from 0.001 to 1 μm, more particularly preferably from 0.01 to 0.5 μm or 0.1 to 1 μm, and further more particularly preferably from 0.01 to 0.3 μm.

In the present invention, the average particle size can be determined from the particle size distribution measured by a static light scattering technique such as laser diffractometry. The particle size distribution is weighted by the volume of each powder while using laser diffractometry or the like to give the distribution. Unless otherwise specified, "average particle size" in the present invention refers to D50 (the diameter at which the larger particle size and the smaller particle size are 50% each when powder is divided into two parts based on their particle size; this is also called the median diameter).

In the aqueous suspension composition of the present invention, sirolimus or a salt thereof with a small average particle size may be purchased commercially. However, it can also be produced in various methods. For example, sirolimus or a salt thereof with a desired average particle size can be obtained by pulverization using a commonly used

pulverizer. There are two main types of pulverization methods, for example, dry pulverization and wet pulverization. Sirolimus or a salt thereof with a desired average particle size can be obtained by using appropriately any of different types of pulverizers such as a ball mill, a bead mill. a pin mill, a jet mill, or a hammer mill. The method of pulverizing sirolimus or a salt thereof contained in the aqueous suspension composition of the present invention is not particularly limited. However, pulverization with a bead mill is preferable, and wet pulverization is preferred. For example, at the time of preparation, sirolimus or a salt thereof and respective components optionally added as necessary are partially dissolved or suspended in purified water. Then, the mixture is subjected to wet pulverization. In this way, the aqueous suspension composition containing sirolimus or a salt thereof with a desired average particle size can be obtained.

In the aqueous suspension composition of the present invention, for example, a surfactant can be added to maintain dispersibility and redispersibility and to suppress agglomeration. Any surfactant that can be used as a pharmaceutical additive may be added appropriately as a surfactant in the case of adding the surfactant in the aqueous suspension composition of the present invention. Examples include a cationic surfactant, an anionic surfactant, an amphoteric surfactant, and a nonionic surfactant. A hydrate or solvate thereof is also acceptable.

Examples of the cationic surfactant include: an amine salt such as an alkylamine salt, an alkylamine polyoxyethylene adduct, a fatty acid triethanolamine monoester, an acylaminoethyldiethylamine salt, a fatty acid polyamine condensate, alkylimidazoline, 1-acylaminoethyl-2-alkylimidazoline, 1-hydroxylethyl-2-alkylimidazoline; and an ammonium salt such as benzalkonium chloride, benzethonium chloride, chlorhexidine gluconate.

Examples of the anionic surfactant include: sulfonic acid or a salt thereof such as alkylbenzene sulfonate, α-olefin sulfonate, α-sulfo fatty acid ester salt; a sulfuric acid ester or a salt thereof such as an alkyl sulfuric acid ester salt, a polyoxyethylene alkyl sulfuric acid ester salt; and phosphoric acid or a salt thereof such as polyoxyethylene alkyl ether phosphate. Specific examples of polyoxyethylene alkyl ether phosphoric acid or a salt thereof include: polyoxyethylene alkyl (12-15) ether phosphoric acid, sodium polyoxyethylene cetyl ether phosphate, polyoxyethylene lauryl ether phosphoric acid, sodium polyoxyethylene lauryl ether phosphate, polyoxyethylene oleyl ether phosphoric acid, and sodium polyoxyethylene oleyl ether phosphate.

Examples of the nonionic surfactant include: a polyoxyethylene fatty acid ester such as polyoxyl 40 stearate, polyoxyl 45 stearate, polyoxyl 55 stearate; a polyoxyethylene sorbitan fatty acid ester such as polysorbate 80, polysorbate 60, polysorbate 40, polyoxyethylene sorbitan monolaurate, polyoxyethylene sorbitan trioleate, polysorbate 65; polyoxyethylene hydrogenated castor oil such as polyoxyethylene hydrogenated castor oil 10, polyoxyethylene hydrogenated castor oil 40, polyoxyethylene hydrogenated castor oil 50, polyoxyethylene hydrogenated castor oil 60; polyoxyl castor oil such as polyoxyl 5 castor oil, polyoxyl 9 castor oil, polyoxyl 15 castor oil, polyoxyl 35 castor oil (also called "CO-35"), polyoxyl 40 castor oil; polyoxyethylene polyoxypropylene glycol such as polyoxyethylene (160) polyoxypropylene (30) glycol, polyoxyethylene (42) polyoxypropylene (67) glycol, polyoxyethylene (54) polyoxypropylene (39) glycol, polyoxyethylene (196) polyoxypropylene (67) glycol, polyoxyethylene (20) polyoxypropylene (20) glycol; a sucrose fatty acid ester such as sucrose stearic acid ester; and tocopherol polyethylene glycol 1000 succinic acid ester (vitamin E TPGS).

The surfactant in the present invention is preferably a nonionic surfactant, more preferably a polyoxyethylene fatty acid ester, a polyoxyethylene sorbitan fatty acid ester, polyoxyethylene hydrogenated castor oil, or polyoxyl castor oil, still more preferably polyoxyl 40 stearate, polysorbate 80, polysorbate 60, polysorbate 40, polyoxyethylene sorbitan monolaurate, polyoxyethylene sorbitan trioleate, polysorbate 65, polyoxyethylene hydrogenated castor oil 10, polyoxyethylene hydrogenated castor oil 40, polyoxyethylene hydrogenated castor oil 50, polyoxyethylene hydrogenated castor oil 60, polyoxyl 5 castor oil, polyoxyl 9 castor oil, polyoxyl 15 castor oil, polyoxyl 35 castor oil, or polyoxyl 40 castor oil, and particularly preferably polyoxyl 40 stearate, polysorbate 80, or polyoxyl 35 castor oil. In addition, an anionic surfactant is preferable. Polyoxyethylene alkyl ether phosphate is more preferable. Sodium polyoxyethylene cetyl ether phosphate, sodium polyoxyethylene lauryl ether phosphate, or sodium polyoxyethylene oleyl ether phosphate is still more preferable. Sodium polyoxyethylene cetyl ether phosphate is particularly preferable.

In the case of adding a surfactant in the aqueous suspension composition of the present invention, two or more surfactants may be used together.

When a surfactant is added in the aqueous suspension composition of the present invention, the content of the surfactant can be adjusted appropriately according to such as the content of sirolimus or a salt thereof and/or the type of the surfactant and the like. For example, from the viewpoint of maintaining dispersibility and redispersibility in the aqueous suspension composition and suppressing agglomeration of the suspension, the lower limit is preferably 0.0001% (w/v) or more than 0.0001% (w/v), or preferably 0.001% (w/v) or more than 0.001% (w/v). The range is preferably from 0.0001 to 5% (w/v), more preferably from 0.001 to 2% (w/v), from 0.001 to 1% (w/v), or from 0.002 to 1% (w/v), still more preferably from 0.005 to 1% (w/v) or from 0.005 to 0.5% (w/v), further preferably from 0.01 to 1% (w/v) or from 0.01 to 0.5% (w/v), and particularly preferably from 0.01 to 0.1% (w/v).

Meanwhile, the content ratio of a surfactant to sirolimus or a salt thereof in the aqueous suspension composition of the present invention can be adjusted appropriately depending on the type of the surfactant and the like. Here, for example, from the viewpoint of maintaining dispersibility and redispersibility in the aqueous suspension composition and suppressing agglomeration, the lower limit of the content of the surfactant based on 1 part by weight of sirolimus or a

salt thereof is preferably more than 0.01 parts by weight, and the upper limit is 100 parts by weight. In addition, the range is preferably from more than 0.01 to 100 parts by weight or from 0.02 to 100 parts by weight, more preferably from 0.05 to 50 parts by weight, still more preferably from 0.1 to 10 parts by weight, and particularly preferably from 0.5 to 2 parts by weight. In addition, also preferred is from 0.1 to 0.5 parts by weight, from 0.1 to 1 part by weight, from 0.5 to 1 part by weight, from 1 to 5 parts by weight, from 1 to 10 parts by weight, or from 5 to 10 parts by weight.

A pharmaceutical additive(s) may be further optionally used in the aqueous suspension composition of the present invention. Specifically, it is possible to add, for example, a dispersant, a buffering agent, a tonicity agent, a stabilizer, an antioxidant, a preservative, and/or a pH adjuster. Each of them may be used alone, or two or more kinds may be used appropriately in combination, and the suitable amount thereof may be added.

Any dispersant that can be used as a pharmaceutical additive may be added appropriately as a dispersant in the case of adding the dispersant in the aqueous suspension composition of the present invention. Examples of the dispersant include: a cellulosic polymer such as methylcellulose, ethylcellulose, hydroxymethylcellulose, hydroxyethylcellulose (also called "HEC"), hydroxypropylcellulose, hydroxyethylmethylcellulose, hydroxypropylmethylcellulose (also called "HPMC" or "hypromellose"), carboxymethyl cellulose (also called "CMC"), sodium carboxymethyl cellulose (also called "CMC-sodium"), hydroxypropylmethylcellulose acetate succinate, hydroxypropylmethylcellulose phthalate, carboxymethylethylcellulose, cellulose acetate phthalate; polyvinylpyrrolidone (also called "PVP"); a polyalcohol such as polyvinyl alcohol (also called "PVA"), polyethylene glycol; a carboxyvinyl polymer; and mucopolysaccharides such as sodium hyaluronate (also called "HA"), chondroitin sulfate. A hydrate or solvate thereof is also acceptable.

As a dispersant in the present invention, preferred is a cellulosic polymer, polyvinylpyrrolidone, a polyalcohol, or mucopolysaccharides. More preferred is a cellulosic polymer. Still more preferred is methylcellulose, ethylcellulose, hydroxymethylcellulose, hydroxyethylcellulose, hydroxypropylcellulose, hydroxyethylmethylcellulose, hydroxypropylmethylcellulose, carboxymethyl cellulose, sodium carboxymethylcellulose, hydroxypropylmethylcellulose acetate succinate, hydroxypropylmethylcellulose phthalate, or carboxymethylethylcellulose. Further preferred is hydroxyethylcellulose, hydroxypropylmethylcellulose, or sodium carboxymethylcellulose. Particularly preferred is hydroxypropylmethylcellulose or sodium carboxymethylcellulose.

In the case of adding a dispersant in the aqueous suspension composition of the present invention, two or more dispersants may be used together.

The content of dispersant in the case of adding the dispersant in the aqueous suspension composition of the present invention may be adjusted appropriately depending on the type of the dispersant and the like, and is preferably from 0.0001 to 0.1% (w/v) and more preferably from 0.0001 to 0.01% (w/v). Also, more preferred is from 0.0001 to 0.001 % (w/v), from 0.0003 to 0.001% (w/v), or from 0.001 to 0.01% (w/v).

Any buffering agent that can be used as a pharmaceutical additive may be added appropriately as a buffering agent in the case of adding the buffering agent in the aqueous suspension composition of the present invention. Examples of the buffering agent include trometamol, phosphoric acid or a salt thereof, boric acid or a salt thereof, carbonic acid or a salt thereof, and an organic acid or a salt thereof. A hydrate or solvate thereof is also acceptable.

Examples of phosphoric acid or a salt thereof include phosphoric acid, trisodium phosphate, sodium dihydrogen phosphate, sodium hydrogen phosphate (disodium hydrogen phosphate), tripotassium phosphate, potassium dihydrogen phosphate, and dipotassium hydrogen phosphate.

Examples of boric acid or a salt thereof include boric acid, sodium borate, and potassium borate.

Examples of carbonic acid or a salt thereof include sodium carbonate and sodium hydrogen carbonate.

Examples of an organic acid or a salt thereof include citric acid, acetic acid, ε-aminocaproic acid, gluconic acid, fumaric acid, lactic acid, ascorbic acid, succinic acid, maleic acid, malic acid, amino acids, and a sodium or potassium salt thereof.

In the case of adding a buffering agent in the aqueous suspension composition of the present invention, two or more buffering agents may be used together.

The content of buffering agent in the case of adding the buffering agent in the aqueous suspension composition of the present invention may be adjusted appropriately depending on the type of the buffering agent and the like, and is preferably from 0.001 to 5% (w/v), more preferably from 0.01 to 2% (w/v), still more preferably from 0.05 to 1 % (w/v), and particularly preferably from 0.05 to 0.5% (w/v). Also, more preferred is from 0.05 to 0.1% (w/v), from 0.05 to 0.2% (w/v), from 0.1 to 0.5% (w/v), or from 0.1 to 0.3% (w/v).

Any tonicity agent that can be used as a pharmaceutical additive may be added appropriately as a tonicity agent in the case of adding the tonicity agent in the aqueous suspension composition of the present invention. Examples of the tonicity agent include an ionic tonicity agent and a nonionic tonicity agent. A hydrate or solvate thereof is also acceptable.

Examples of the ionic tonicity agent include sodium chloride, potassium chloride, calcium chloride, and magnesium chloride.

Examples of the nonionic tonicity agent include glycerin, concentrated glycerin, propylene glycol, polyethylene glycol, sorbitol, mannitol, trehalose, maltose, sucrose, and xylitol.

In the case of adding a tonicity agent in the aqueous suspension composition of the present invention, two or more tonicity agents may be used together.

The content of tonicity agent in the case of adding the tonicity agent in the aqueous suspension composition of the present invention may be adjusted appropriately depending on the type of the tonicity agent and the like, and is preferably from 0.001 to 10% (w/v), more preferably from 0.01 to 5% (w/v), still more preferably from 0.1 to 3% (w/v), and particularly preferably from 0.5 to 3% (w/v).

Any stabilizer that can be used as a pharmaceutical additive may be added appropriately as a stabilizer in the case of adding the stabilizer in the aqueous suspension composition of the present invention. Examples of the stabilizer include edetic acid or a salt thereof. A hydrate or solvate thereof is also acceptable.

Examples of edetic acid or a salt thereof include edetic acid and sodium edetate.

In the case of adding a stabilizer in the aqueous suspension composition of the present invention, two or more stabilizers may be used together.

The content of stabilizer in the case of adding the stabilizer in the aqueous suspension composition of the present invention may be adjusted appropriately depending on the type of the stabilizer and the like, and is preferably from 0.001 to 1% (w/v), more preferably from 0.005% to 0.1% (w/v), and still more preferably from 0.01 to 0.05% (w/v).

Any antioxidant that can be used as a pharmaceutical additive may be added appropriately as an antioxidant in the case of adding the antioxidant in the aqueous suspension composition of the present invention. Examples of the antioxidant include ascorbic acid, tocopherol, dibutylhydroxytoluene, and sodium sulfite. A hydrate or solvate thereof is also acceptable.

In the case of adding an antioxidant in the aqueous suspension composition of the present invention, two or more antioxidants may be used together.

The content of antioxidant in the case of adding the antioxidant in the aqueous suspension composition of the present invention may be adjusted appropriately depending on the type of the antioxidant and the like, and is preferably from 0.001 to 5% (w/v), more preferably from 0.01 % to 3% (w/v), and still more preferably from 0.1 to 2% (w/v).

Any preservative that can be used as a pharmaceutical additive may be added appropriately as a preservative in the case of adding the preservative in the aqueous suspension composition of the present invention. Examples of the preservative include invert soaps, parabens, an organic acid or a salt thereof, chlorobutanol, and silver nitrate. A hydrate or solvate thereof is also acceptable.

Examples of the invert soaps include benzalkonium chloride, benzalkonium bromide, benzethonium chloride, benzethonium bromide, chlorhexidine gluconate, and chlorhexidine hydrochloride.

Examples of the parabens include methyl paraoxybenzoate, ethyl paraoxybenzoate, propyl paraoxybenzoate, and butyl paraoxybenzoate.

Examples of the organic acid or the salt thereof include sorbic acid or a salt thereof and sodium dehydroacetate. Examples of the sorbic acid or the salt thereof among them include sodium sorbate and potassium sorbate.

In the case of adding a preservative in the aqueous suspension composition of the present invention, two or more preservatives may be used together.

The content of preservative in the case of adding the preservative in the aqueous suspension composition of the present invention may be adjusted appropriately depending on the type of the preservative and the like. The content of preservative may be an amount at which there is no harmful effect on safety. The upper limit is, for example, 1 % (w/v), preferably 1% (w/v) or less, more preferably 0.5% (w/v) or less, still more preferably 0.1 % (w/v) or less, and further preferably 0.01% (w/v) or less. In addition, the amount may be permitted if the preservative action can be elicited. The lower limit is, for example, 0.0001 % (w/v), preferably 0.0001% (w/v) or more, and more preferably 0.001% (w/v) or more. The content of preservative is preferably from 0.0001 to 1% (w/v), more preferably from 0.001 to 0.5% (w/v), and still more preferably from 0.001 to 0.1% (w/v).

Any pH adjuster that can be used as a pharmaceutical additive may be added appropriately as a pH adjuster in the case of adding the pH adjuster in the aqueous suspension composition of the present invention, for example, it may be acid or base. Examples of the acid include hydrochloric acid, phosphoric acid, citric acid, and acetic acid. Examples of the base include sodium hydroxide, potassium hydroxide, sodium carbonate, and sodium hydrogen carbonate. A hydrate or solvate thereof is also acceptable.

In the case of adding a pH adjuster in the aqueous suspension composition of the present invention, two or more pH adjusters may be used together.

The pH of the aqueous suspension composition in the present invention is acceptable as long as it is within a range acceptable for pharmaceutical use. However, from the viewpoint of stability of the aqueous suspension composition, the pH is preferably near 5. The pH is more preferably from 4 to 6, still more preferably from 4.0 to 6.0, further preferably from 4.1 to 5.9, particularly preferably from 4.5 to 5.5, further particularly preferably from 4.7 to 5.3, and particularly preferably 5.0. More specific examples include 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, and 6.0.

The osmotic pressure rate of the aqueous suspension composition of the present invention is acceptable as

long as it is within a range acceptable for pharmaceutical use. For example, the range is from 0.5 to 2.0, preferably from 0.7 to 1.6, more preferably from 0.8 to 1.4, and still more preferably from 0.9 to 1.2.

In the present invention, the aqueous suspension composition is a suspension composition comprising water as a solvent. The content of water in the aqueous suspension composition is preferably 80 mass% or more, more preferably 90 mass% or more, and still more preferably 95 mass% or more.

In the present invention, the aqueous suspension composition is a dispersion system in which solid particles are dispersed in a liquid. The components of the aqueous suspension composition are not necessarily dissolved at all or may be partially dissolved. In addition, when the aqueous suspension composition is allowed to stand, the solid particles may be in a precipitated state. Even if the solid particles are in a precipitated state, the composition may be shaken, as a result, the solid particles can be reconstituted in a dispersion system (also referred to as re-dispersion). The aqueous suspension composition of the present invention doesn't include a liquid in which the components are completely dissolved.

Unless otherwise indicated, an aqueous suspension composition of the present invention may comprise an active ingredient that is other than sirolimus or a salt thereof and is used for eye drops. However, sirolimus or a salt thereof may be comprised as a sole active ingredient.

An aqueous suspension composition of the present invention may be administered to a patient, for example, orally or parenterally, and preferably parenterally. The aqueous suspension composition of the present invention is used for ophthalmology. Thus, the parenteral administration is preferably topical ocular administration. More preferred is ocular instillation administration, subconjunctival administration, intraconjunctival administration, sub-Tenon administration, or dermal administration. Still more preferred is ocular instillation administration from the viewpoint of low invasive characteristics. In addition, the dermal administration is preferably eyelid skin administration.

The aqueous suspension composition of the present invention is for ophthalmology. Thus, the aqueous suspension composition of the present invention can be used as an ophthalmic preparation. The dosage form is not particularly limited as long as it can be used as a pharmaceutical agent. Examples of the dosage form include an eye drop, ointment, cream, gel, a transdermal formulation, a patch, and an injection. Particularly preferred is an eye drop.

A suitable amount of the aqueous suspension composition of the present invention is preferably administered once a day or separately administered two to six times a day. In the case where the aqueous suspension composition is an eye drop, in particular, one dose of one or two drops per eye is applied to an eye(s) once a day or separately applied to an eye(s) two to four times a day. One dose of one drop per eye is still more preferably applied to an eye(s) once a day or separately applied to an eye(s) two to four times a day. One dose of one drop per eye is more preferably applied to an eye(s) once a day or separately applied to an eye(s) two times a day. One dose of one drop per eye is particularly preferably applied to an eye(s) once a day. Note that an aqueous suspension composition of the present invention may be separately applied to an eye(s) two to four times a day. In this case, the ocular administration interval may be at least one hour or longer, preferably two hours or longer, and more preferably three hours or longer. One drop is usually from approximately 0.01 to approximately 0.1 mL, preferably from approximately 0.015 to approximately 0.07 mL, more preferably from approximately 0.02 to approximately 0.05 mL, and particularly preferably approximately 0.03 mL.

A container containing an aqueous suspension composition of the present invention is not particularly limited as long as it is a container generally and commonly used as a container containing a medicament. In the case where the aqueous suspension composition is an eye drop, in particular, any of a multi-dose type container, a single-use unit-dose type container, or a PFMD (Preservative Free Multi Dose) container is acceptable. Note that the material for the container is not particularly limited as long as the container can be generally and commonly used for an eye drop. However, the container is preferably a resin container. For example, it is possible to use a container made of polyethylene (PE), polypropylene (PP), polyethylene terephthalate (PET), polybutylene terephthalate (PBT), polypropylene-polyethylene copolymer, polyvinyl chloride, acryl, polystyrene, polycyclic olefin copolymer, or the like. Meanwhile, if the material for the resin container is, for example, polyethylene, it is possible to use a container made of low-density polyethylene (LDPE), medium-density polyethylene (MDPE), high-density polyethylene (HDPE), or the like, as the polyethylene is classified according to its density.

An aqueous suspension composition of the present invention may be prepared by a generalized method. For example, it may be prepared as the following: sirolimus or a salt thereof and respective components optionally added are partially dissolved or suspended in purified water; the mixture is optionally subjected to wet pulverization; and the osmotic pressure, the pH, and others may be each adjusted within a given range.

The aqueous suspension composition of the present invention is used for ophthalmology as described above. For example, the aqueous suspension composition is useful for the treatment and/or prevention of ocular disease, thus, for example, it is an aqueous suspension composition for use in the treatment and/or prevention of ocular disease. The aqueous suspension composition of the present invention may be used for the treatment and/or prevention of ocular disease. In this case, a disease of interest is not particularly limited and may be anterior eye disease or posterior ocular disease. Here, the aqueous suspension composition of the present invention may be administered topically as a low-invasive eye drop. Accordingly, in particular, it is preferably used for the treatment and/or prevention of anterior eye disease. Examples of specific disease include keratitis, corneal endothelial disorder (corneal endotheliitis, macular corneal dystrophy, Fuchs endothelial corneal dystrophy, bullous keratopathy), keratoconjunctivitis, conjunctivitis, blepharitis, meibomian gland dysfunction (also called "MGD"), ocular dry eye syndrome (also called "dry eye"), Sjogren’s syndrome, allergic conjunctivitis, uveitis, endophthalmitis, graft-versus-host-disease (also called "GVHD"), postoperative inflammation of the anterior eye segment, inflammation due to rejection of ocular tissue transplantation, and corneal (bacterial, fungal, amoebic) infection. In the case of using an aqueous suspension composition of the present invention for the treatment of ocular disease, typically, a therapeutically effective amount of the aqueous suspension composition of the present invention is administered to and used for a patient.

In the present invention, the "patient" is not limited to only a human, and means, for example, a dog, a cat, or a horse. A patient in the present invention is preferably a mammal and more preferably a human. In the present invention, the "therapeutically effective amount" refers to an amount at which therapeutic efficacy on the disease or its symptom can be exerted or the progress of the disease or its symptom can be delayed, more than in untreated subjects.

One aspect of the present invention is a method of suppressing agglomeration of sirolimus or a salt thereof in the aqueous suspension composition comprising sirolimus or a salt thereof and a surfactant, comprising adjusting the pH of the aqueous suspension composition to 4 to 6 and/or setting the average particle size of sirolimus or a salt thereof in the aqueous suspension composition to 45 μm or less. The method of the present invention, as described above, is characterized in that in the aqueous suspension composition comprising sirolimus or a salt thereof and a surfactant, the pH is adjusted to 4 to 6 and/or the average particle size of sirolimus or a salt thereof is set to 45 μm or less. Note that the detailed description about the above aqueous suspension composition of the present invention is applicable to the method of suppressing agglomeration of sirolimus or a salt thereof of the present invention.

One aspect of the present invention is a method of suppressing decomposition of sirolimus in an aqueous suspension composition comprising sirolimus or a salt thereof and a surfactant, comprising adjusting the pH of the aqueous suspension composition to 4 to 6. The method of the present invention, as described above, is characterized in that in the aqueous suspension composition comprising sirolimus or a salt thereof and a surfactant, the pH is adjusted to 4 to 6. Note that the detailed description about the above aqueous suspension composition of the present invention is applicable to the method of suppressing decomposition of sirolimus of the present invention.

One aspect of the present invention is a method of treating ocular disease, comprising administering to a patient in need of the treatment a therapeutically effective amount of the aqueous suspension composition of the present invention. The ocular disease treatment method of the present invention, as described above, is characterized in that a therapeutically effective amount of the aqueous suspension composition of the present invention is administered to a patient in need of the treatment of ocular disease. In the ocular disease treatment method of the present invention, the ocular disease is preferably anterior eye disease. Note that the detailed description about the above aqueous suspension composition of the present invention is applicable to the ocular disease treatment method of the present invention.

One aspect of the present invention is use of the aqueous suspension composition of the present invention for the manufacture of a medicament for treating and/or preventing ocular disease. The use of the present invention, as described above, is characterized in that the aqueous suspension composition of the present invention is used for the manufacture of a medicament for treating and/or preventing ocular disease. In the use of the present invention, the ocular disease is preferably anterior eye disease. Note that the detailed description about the above aqueous suspension composition of the present invention is applicable to the use of the present invention.

## EXAMPLES

Hereinafter, Preparation Examples and Test Examples will be illustrated. However, they are used for better understanding of the present invention. Thus, the scope of the present invention should not be limited by them.

### Preparation Examples

Representative Preparation Examples of the present invention will be shown below. Note that the added amount of each component in the below-described Preparation Examples is the content in 100 mL of each preparation.

### Preparation Example 1

| | |
|---|---|
| Sirolimus | 0.01 g |
| Polysorbate 80 | 0.01 g |

(continued)

| | |
|---|---|
| Hypromellose | 0.0001 g |
| Sodium citrate hydrate | 0.1 g |
| Sodium edetate hydrate | 0.01 g |
| Concentrated glycerin | 2.0 g |
| Benzalkonium chloride | 0.001 g |
| Sodium hydroxide/Dilute hydrochloric acid | q.s. |
| Purified water | q.s. |
| pH | 5.0 |

Preparation Example 2

| | |
|---|---|
| Sirolimus | 0.1 g |
| Polysorbate 80 | 0.01 g |
| CMC-sodium | 0.01 g |
| Sodium citrate hydrate | 0.1 g |
| Sodium edetate hydrate | 0.01 g |
| Concentrated glycerin | 1.5 g |
| Benzalkonium chloride | 0.001 g |
| Sodium hydroxide/Dilute hydrochloric acid | q.s. |
| Purified water | q.s. |
| pH | 5.0 |

Preparation Example 3

| | |
|---|---|
| Sirolimus | 0.05 g |
| Polyoxyl 40 stearate | 0.1 g |
| CMC-sodium | 0.001 g |
| Sodium hydrogen phosphate hydrate | 0.05 g |
| Sodium edetate hydrate | 0.02 g |
| Sodium chloride | 0.8 g |
| Sodium hydroxide/Dilute hydrochloric acid | q.s. |
| Purified water | q.s. |
| pH | 5.0 |

Preparation Example 4

| | |
|---|---|
| Sirolimus | 0.2 g |
| Polyoxyl 40 stearate | 0.2 g |
| Hypromellose | 0.0005 g |
| Sodium hydrogen phosphate hydrate | 0.05 g |
| Sodium edetate hydrate | 0.075 g |
| Sodium chloride | 1.2 g |
| Sodium hydroxide/Dilute hydrochloric acid | q.s. |
| Purified water | q.s. |
| pH | 5.0 |

Preparation Example 5

| | |
|---|---|
| Sirolimus | 0.1 g |
| Polysorbate 80 | 0.005 g |
| CMC-sodium | 0.01 g |
| Sodium citrate hydrate | 0.05 g |
| Sodium edetate hydrate | 0.02 g |
| Sodium chloride | 0.9 g |
| Sodium hydroxide/Dilute hydrochloric acid | q.s. |
| Purified water | q.s. |
| pH | 5.0 |

Preparation Example 6

| | |
|---|---|
| Sirolimus | 0.1 g |
| Polysorbate 80 | 0.05 g |
| CMC-sodium | 0.01 g |
| Sodium citrate hydrate | 0.1 g |
| Sodium edetate hydrate | 0.01 g |
| Concentrated glycerin | 1.8 g |
| Sodium hydroxide/Dilute hydrochloric acid | q.s. |
| Purified water | q.s. |
| pH | 5.0 |

Preparation Example 7

| | |
|---|---|
| Sirolimus | 0.03 g |
| Polysorbate 80 | 0.01 g |
| CMC-sodium | 0.005 g |
| Sodium hydrogen phosphate hydrate | 0.1 g |
| Sodium edetate hydrate | 0.01 g |
| Sodium chloride | 0.75 g |
| Benzalkonium chloride | 0.001 g |
| Sodium hydroxide/Dilute hydrochloric acid | q.s. |
| Purified water | q.s. |
| pH | 5.0 |

Preparation Example 8

| | |
|---|---|
| Sirolimus | 0.03 g |
| Polysorbate 80 | 0.05 g |
| Hydroxyethylcellulose | 0.01 g |
| Sodium hydrogen phosphate hydrate | 0.1 g |
| Sodium edetate hydrate | 0.01 g |
| Concentrated glycerin | 2.0 g |

(continued)

| | |
|---|---|
| Sodium hydroxide/Dilute hydrochloric acid | q.s. |
| Purified water | q.s. |
| pH | 5.0 |

Preparation Example 9

| | |
|---|---|
| Sirolimus | 0.03 g |
| Polysorbate 80 | 0.1 g |
| Hypromellose | 0.001 g |
| Sodium citrate hydrate | 0.05 g |
| Concentrated glycerin | 1.8 g |
| Benzalkonium chloride | 0.001 g |
| Sodium hydroxide/Dilute hydrochloric acid | q.s. |
| Purified water | q.s. |
| pH | 5.0 |

Preparation Example 10

| | |
|---|---|
| Sirolimus | 0.03 g |
| Polysorbate 80 | 0.03 g |
| Hypromellose | 0.0003 g |
| Sodium citrate hydrate | 0.1 g |
| Sodium edetate hydrate | 0.01 g |
| Sodium chloride | 0.85 g |
| Sodium hydroxide/Dilute hydrochloric acid | q.s. |
| Purified water | q.s. |
| pH | 5.0 |

Preparation Example 11

| | |
|---|---|
| Sirolimus | 0.1 g |
| Polyoxyl 35 castor oil | 0.05 g |
| Hydroxyethylcellulose | 0.001 g |
| Sodium citrate hydrate | 0.1 g |
| Sodium edetate hydrate | 0.01 g |
| Sodium chloride | 0.7 g |
| Benzalkonium chloride | 0.001 g |
| Sodium hydroxide/Dilute hydrochloric acid | q.s. |
| Purified water | q.s. |
| pH | 5.0 |

Preparation Example 12

| | |
|---|---|
| Sirolimus | 0.1 g |
| Polyoxyl 35 castor oil | 0.05 g |
| Polyvinylpyrrolidone | 0.01 g |
| Sodium hydrogen phosphate hydrate | 0.05 g |
| Sodium edetate hydrate | 0.02 g |
| Concentrated glycerin | 1.4 g |
| Benzalkonium chloride | 0.002 g |
| Sodium hydroxide/Dilute hydrochloric acid | q.s. |
| Purified water | q.s. |
| pH | 5.0 |

Preparation Example 13

| | |
|---|---|
| Sirolimus | 0.1 g |
| Polyoxyl 35 castor oil | 0.1 g |
| Hypromellose | 0.005 g |
| Sodium hydrogen phosphate hydrate | 0.1 g |
| Sodium edetate hydrate | 0.05 g |
| Concentrated glycerin | 1.2 g |
| Sodium hydroxide/Dilute hydrochloric acid | q.s. |
| Purified water | q.s. |
| pH | 5.0 |

Preparation Example 14

| | |
|---|---|
| Sirolimus | 0.1 g |
| Polysorbate 80 | 0.05 g |
| Polyvinyl alcohol | 0.01 g |
| Sodium hydrogen phosphate hydrate | 0.05 g |
| Sodium edetate hydrate | 0.03 g |
| Concentrated glycerin | 1.5 g |
| Sodium hydroxide/Dilute hydrochloric acid | q.s. |
| Purified water | q.s. |
| pH | 5.0 |

Preparation Example 15

| | |
|---|---|
| Sirolimus | 0.1 g |
| Polysorbate 80 | 0.1 g |
| Hypromellose | 0.0003 g |
| Sodium citrate hydrate | 0.1 g |
| Sodium edetate hydrate | 0.01 g |
| Sodium chloride | 0.85 g |
| Sodium hydroxide/Dilute hydrochloric acid | q.s. |
| Purified water | q.s. |
| pH | 5.0 |

Preparation Example 16

| | |
|---|---|
| Sirolimus | 0.1 g |
| Polysorbate 80 | 0.1 g |
| Hypromellose | 0.01 g |
| Sodium citrate hydrate | 0.05 g |
| Sodium edetate hydrate | 0.03 g |
| Sodium chloride | 0.7 g |
| Silver nitrate | 0.005 g |
| Sodium hydroxide/Dilute hydrochloric acid | q.s. |
| Purified water | q.s. |
| pH | 5.0 |

Preparation Example 17

| | |
|---|---|
| Sirolimus | 0.1 g |
| Polyoxyl 35 castor oil | 0.05 g |
| Hypromellose | 0.001 g |
| Sodium citrate hydrate | 0.1 g |
| Sodium edetate hydrate | 0.03 g |
| Concentrated glycerin | 1.5 g |
| Chlorobutanol | 0.01 g |
| Sodium hydroxide/Dilute hydrochloric acid | q.s. |
| Purified water | q.s. |
| pH | 5.0 |

Preparation Example 18

| | |
|---|---|
| Sirolimus | 0.1 g |
| Polysorbate 80 | 0.05 g |
| CMC-sodium | 0.01 g |
| Sodium hydrogen phosphate hydrate | 0.1 g |
| Sodium edetate hydrate | 0.01 g |
| Sodium chloride | 0.9 g |
| Chlorobutanol | 0.01 g |
| Sodium hydroxide/Dilute hydrochloric acid | q.s. |
| Purified water | q.s. |
| pH | 5.0 |

Preparation Example 19

| | |
|---|---|
| Sirolimus | 0.3 g |
| Polysorbate 80 | 0.5 g |
| Hydroxyethylcellulose | 0.01 g |
| Sodium hydrogen phosphate hydrate | 0.085 g |

(continued)

| | |
|---|---|
| Sodium edetate hydrate | 0.01 g |
| Concentrated glycerin | 2.0 g |
| Sodium hydroxide/Dilute hydrochloric acid | q.s. |
| Purified water | q.s. |
| pH | 5.0 |

Preparation Example 20

| | |
|---|---|
| Sirolimus | 0.5 g |
| Polysorbate 80 | 0.5 g |
| CMC-sodium | 0.01 g |
| Sodium hydrogen phosphate hydrate | 0.085 g |
| Sodium edetate hydrate | 0.01 g |
| Concentrated glycerin | 1.8 g |
| Sodium hydroxide/Dilute hydrochloric acid | q.s. |
| Purified water | q.s. |
| pH | 5.0 |

Test Examples

1. Stability Test

(1) To Prepare Test Preparation

In order to obtain the given concentration of each component contained, crushed sirolimus (with an average particle size: 15 μm), polysorbate 80, hypromellose TC5 (registered trademark), concentrated glycerin, sodium dihydrogen phosphate hydrate, sodium edetate hydrate, and purified water were mixed. Next, a pH adjuster (hydrochloric acid and/or sodium hydroxide) and purified water were added thereto to have a total volume of 100 mL. In this way, a test preparation of composition 1 (pH 3.0; a suspension) was prepared. In addition, the same method as for the test preparation of composition 1 was used, except for the pH adjustment, to prepare compositions 2 to 4 (pH 5.0 to pH 9.0; any of them was a suspension). The concentration of each component contained in each test preparation was as shown in Table 1.

[Table 1]

| Test Preparation [% (w/v)] | Composition 1 | Composition 2 | Composition 3 | Composition 4 |
|---|---|---|---|---|
| Sirolimus | 0.01 | 0.01 | 0.01 | 0.01 |
| Polysorbate 80 | 0.1 | 0.1 | 0.1 | 0.1 |
| Hypromellose | 0.01 | 0.01 | 0.01 | 0.01 |
| Sodium edetate hydrate | 0.01 | 0.01 | 0.01 | 0.01 |
| Concentrated glycerin | q.s. | | | |
| Sodium dihydrogen phosphate hydrate | q.s. | | | |
| pH adjuster | q.s. | | | |
| Purified water | q.s. | | | |
| pH | 3.0 | 5.0 | 7.0 | 9.0 |

(2) Test Method

Here, 5 mL of each of the test preparations of compositions 1 to 4 was filled into a sterile container, and tightly

sealed. Each container was stored for 4 weeks in an incubator at a temperature of 60°C (ambient humidity) or at a temperature of 40°C and a humidity of 20%. At the time of immediately after filling and after 4 weeks of storage, dispersing well the solid particles in each composition, a small amount was sampled from the composition. Then, a common procedure using ultra-high performance liquid chromatography (UPLC) was used to measure the residual amount of sirolimus contained in the composition. Further, the residual rate of sirolimus was calculated. The residual rate of sirolimus was calculated using the equation below.

$$\text{Residual rate (\%)} = 100 \times [(\text{Residual amount of sirolimus after storage})/(\text{Residual amount of sirolimus immediately after filling})].$$

Note that more detailed UPLC measurement conditions were as follows.

[Column] ACQUITY UPLC BEH C18 (1.7 μm; 2.1 mm × 50 mm)
[Guard column] ACQUITY UPLC BEH C18 VangurardPre-column (1.7 μm; 2.1 mm × 5 mm)
[Column temperature] 45°C
[Mobile phase] Gradient with solution A (20 mM ammonium acetate buffer) and solution B (methanol-acetonitrile mixture (1:1))

(3) Test Results and Discussion

Table 2 shows the test results.

[Table 2]

| Test Results | Storage Conditions (Temperature, Humidity) | Composition 1 | Composition 2 | Composition 3 | Composition 4 |
|---|---|---|---|---|---|
| Residual rate (%) | 60°C, ambient | 56.7 | 97.7 | 59.1 | 65.0 |
| | 40°C, 20% | 89.6 | 107.9 | 82.7 | 72.7 |

Table 2 has clearly revealed that the stability of sirolimus-containing composition depends on the pH. In particular, the stability at or near pH 5 has been found to be stable.

2. Agglomeration Evaluation and Stability Test

(1) To Prepare Test Preparation

First, uncrushed sirolimus, polysorbate 80, and purified water were mixed. Next, the mixture was subjected to wet pulverization using a bead mill until the average particle size became 0.50 μm or less. Then, hypromellose TC5 (registered trademark), sodium citrate hydrate, sodium edetate hydrate, and sodium chloride were mixed. After that, a pH adjuster (hydrochloric acid and/or sodium hydroxide) and purified water were added thereto to have a total volume of 100 mL. In this way, a test preparation of composition 5 (pH 3.0; a suspension) was prepared. In addition, the same method as for the test preparation of composition 5 was used, except for the pH adjustment, to prepare compositions 6 to 10 (pH 4.0 to pH 6.0; any of them was a suspension). The same method as for the test preparation of composition 8 was used, except that wet pulverization using a bead mill was conducted until the average particle size reached 0.30 μm or less, to prepare a test preparation of composition 11. Further, the same method as for the test preparation of composition 8 was used, except that no wet pulverization was conducted while crushed sirolimus (with an average particle size: 2.5 μm) was used, to prepare a test preparation of composition 12. The concentration of each component contained in each test preparation was as shown in Table 3.

[Table 3]

| Test Preparation [% (w/v)] | Compo-sition 5 | Composition 6 | Composition 7 | Composition 8 | Composition 9 | Composition 10 | Composition 11 | Composition 12 |
|---|---|---|---|---|---|---|---|---|
| Sirolimus | 0.1 | 0.1 | 0.1 | 0.1 | 0.1 | 0.1 | 0.1 | 0.1 |
| Polysorbate 80 | 0.1 | 0.1 | 0.1 | 0.1 | 0.1 | 0.1 | 0.1 | 0.1 |
| Hypromellose | 0.0003 | 0.0003 | 0.0003 | 0.0003 | 0.0003 | 0.0003 | 0.0003 | 0.0003 |
| Sodium edetate hydrate | 0.01 | 0.01 | 0.01 | 0.01 | 0.01 | 0.01 | 0.01 | 0.01 |
| Sodium citrate hydrate | q.s. | | | | | | | |
| Sodium chloride | q.s. | | | | | | | |
| pH adjuster | q.s. | | | | | | | |
| Purified water | q.s. | | | | | | | |
| pH | 3.0 | 4.0 | 4.5 | 5.0 | 5.5 | 6.0 | 5.0 | 5.0 |
| Estimate of the average particle size of sirolimus used | 0.50 μm or less | | | | | | 0.30 μm or less | 2.5 μm |

(2) Test Method

Here, 5 mL of each of the test preparations of compositions 5 to 12 was filled into a sterile container, and tightly sealed. Each container was stored for 4 weeks in an incubator at a temperature of 60°C (ambient humidity) or stored for 2 weeks at a temperature of 40°C and a humidity of 20%. At the time of immediately after filling and after 4 or 2 weeks of storage, a zeta-potential/particle size measuring system (ELSZ-1000ZS, manufactured by Otsuka Electronics Co., Ltd.) was used to measure the average particle size of each test preparation. Also, a particle size increase rate was calculated. The particle size increase rate was calculated by the equation below.

Particle size increase rate = (Average particle size after storage)/(Average particle size immediately after filling).

Note that, more detailed average particle size measurement conditions were as follows.

[Measurement conditions] Temperature: 25°C, refractive index of solvent: 1.3328, viscosity of solvent: 0.89, scattering intensity: auto, incident light filter: auto
[Cell conditions] Number of integrations: 70 times, dust cut: 10 times
[Analysis conditions] Average particle size analysis: cumulant method, particle size distribution analysis: Marquardt method

As for those stored at a temperature of 60°C (ambient humidity), the residual rate of sirolimus in each test preparation was calculated in substantially the same manner as in the above section "1. Stability Test".
(3) Test Results and Discussion
Table 4 shows the test results. Note that the "-" in the table indicates "Not Determined".

[Table 4]

| Test Results | Storage Conditions (Temperature, Humidity) | Composition 5 | Composition 6 | Composition 7 | Composition 8 | Composition 9 | Composition 10 | Composition 11 | Compo-sition 12 |
|---|---|---|---|---|---|---|---|---|---|
| Average particle size (μm) | Immediately after filling | 0.32 | 0.39 | - | 0.48 | - | 0.38 | 0.17 | 6.40 |
| | 60°C, ambient | 1.05 | 0.70 | - | 0.58 | - | 0.73 | - | - |
| | 40°C, 20% | - | - | - | 0.47 | - | - | 0.17 | 6.40 |
| Particle size increase rate | 60°C, ambient | 3.28 | 1.79 | - | 1.21 | - | 1.92 | - | - |
| | 40°C, 20% | - | - | - | 0.98 | - | - | 1 | 1 |
| Residual rate (%) | 60°C, ambient | 79.9 | 97.9 | 100.2 | 100.3 | 99.5 | 95.9 | - | - |

Table 4 clearly shows that the particle size increase rate depends on the pH. In particular, the minimum was at or near pH 5. The particle size increase rate was found to increase as the pH was deviated from 5. The particle size increase rate can be considered as an indicator that indicates the degree of particle aggregation. Specifically, it has been suggested that agglomeration is unlikely to occur at or near pH 5 and as the pH is deviated from 5, agglomeration during storage is more likely to occur. It has also been suggested that in the case of sirolimus which is used for a test preparation with a large particle size (composition 12), agglomeration is likely to occur at the preparation time point of the test preparation. On the other hand, if the pH is at or near 5 even when the particle size is large at the preparation time point, agglomeration during storage is found to be unlikely to occur.

In addition, the results of Table 4 and Table 2 in the section "1. Stability Test" have demonstrated that the sirolimus-containing aqueous suspension compositions are stable at or near pH 5 and their agglomeration is unlikely to occur.

3. Agglomeration Test

(1) To Prepare Test Preparation

First, uncrushed sirolimus, each surfactant, and purified water were mixed. Next, the mixture was subjected to wet pulverization using a bead mill until the average particle size became 0.50 μm or less. Then, the other components were mixed. After that, a pH adjuster (hydrochloric acid and/or sodium hydroxide) and purified water were added thereto to have a total volume of 100 mL. In this way, compositions 13 to 24 (any of them was a suspension) ware prepared. The concentration of each component contained in each test preparation was as shown in Table 5 or 6. Note that in Table 5, "MYS40" means polyoxyl 40 stearate and "TCP5" means sodium polyoxyethylene cetyl ether phosphate.

[Table 5]

| Test Preparation [% (w/v)] | Composition 13 | Composition 14 | Composition 15 | Composition 16 | Composition 17 | Composition 18 |
|---|---|---|---|---|---|---|
| Sirolimus | 0.1 | 0.1 | 0.1 | 0.1 | 0.1 | 0.1 |
| Polysorbate 80 | 0.001 | 0.01 | 0.1 | 1 | - | - |
| MYS40 | - | - | - | - | 0.1 | - |
| TCP5 | - | - | - | - | - | 0.1 |
| CMC-sodium | 0.01 | 0.01 | 0.01 | 0.01 | 0.01 | - |
| Sodium edetate hydrate | 0.01 | 0.01 | 0.01 | 0.01 | 0.01 | 0.01 |
| Concentrated glycerin | q.s. | | | | | |
| Sodium citrate hydrate | q.s. | | | | | |
| pH adjuster | q.s. | | | | | |
| Purified water | q.s. | | | | | |
| pH | 5.0 | | | | | |

[Table 6]

| Test Preparation [% (w/v)] | Composition 19 | Composition 20 | Composition 21 | Composition 22 | Composition 23 | Composition 24 |
|---|---|---|---|---|---|---|
| Sirolimus | 1 | 1 | 0.1 | 0.1 | 0.1 | 0.1 |

(continued)

| Test Preparation [% (w/v)] | Composition 19 | Composition 20 | Composition 21 | Composition 22 | Composition 23 | Composition 24 |
|---|---|---|---|---|---|---|
| Polysorbate 80 | 0.01 | 0.1 | 0.1 | 0.1 | 0.001 | 0.01 |
| Hypromellose | 0.001 | 0.001 | - | 0.0001 | 0.001 | 0.001 |
| Sodium edetate hydrate | 0.01 | 0.01 | 0.01 | 0.01 | 0.01 | 0.01 |
| Concentrated glycerin | q.s. | | | | | |
| Sodium citrate hydrate | q.s. | | | | | |
| pH adjuster | q.s. | | | | | |
| Purified water | q.s. | | | | | |
| pH | 5.0 | | | | | |

(2) Test Method

Here, 5 mL of each of the test preparations of compositions 13 to 24 was filled into a sterile container, and tightly sealed. Each container was stored for 4 weeks at a temperature of 40°C and a humidity of 20%. At the time of immediately after filling and after 4 weeks of storage, the same method as in the section "2. Agglomeration Evaluation and Stability Test" was used to measure the average particle size and calculate the particle size increase rate.

(3) Test Results and Discussion

Table 7 and 8 show the test results. Note that the "Unsuitable" in the tables indicates that the test preparation was not stored because it was visually confirmed that a large number of aggregates was formed when the test preparation was prepared.

[Table 7]

| Test Results | Storage Conditions (Temperature, Humidity) | Composition 13 | Composition 14 | Composition 15 | Composition 16 | Composition 17 | Composition 18 |
|---|---|---|---|---|---|---|---|
| Average particle size | Immediately after filling | Unsuitable | 0.45 | 0.38 | 1.41 | 0.36 | 0.38 |
| (μm) | 40°C, 20% | - | 0.91 | 0.39 | 1.04 | 0.42 | 0.42 |
| Particle size increase rate | 40°C, 20% | - | 2.02 | 1.03 | 0.74 | 1.17 | 1.10 |

[Table 8]

| Test Results | Storage Conditions (Temperature, Humidity) | Composition 19 | Composition 20 | Composition 21 | Composition 22 | Composition 23 | Composition 24 |
|---|---|---|---|---|---|---|---|
| Average particle size (μm) | Immediately after filling | Unsuitable | 0.40 | 0.33 | 0.34 | Unsuitable | 0.98 |
| | 40°C, 20% | - | 0.40 | 0.33 | 0.37 | - | 1.19 |
| Particle size increase rate | 40°C, 20% | - | 1 | 1 | 1.09 | - | 1.21 |

As demonstrated in Tables 7 and 8, it was found that in any of the cases where the amount of surfactant was enough low relative to the amount of sirolimus and the content of the surfactant based on 1 part by weight of sirolimus was 0.01 parts by weight (in the cases of compositions 13, 19, and 23), a large number of aggregates was formed. The type of surfactant or the type of dispersant do not largely affect the particle aggregation.

4. Agglomeration Test

(1) To Prepare Test Preparation

First, uncrushed sirolimus, polysorbate 80, hypromellose TC5 (registered trademark), sodium chloride, sodium citrate hydrate, sodium edetate hydrate, and purified water were mixed. Next, a pH adjuster (hydrochloric acid and/or sodium hydroxide) and purified water were added thereto to have a total volume of 100 mL. In this way, a test preparation of composition 25 (a suspension) was prepared. The same method as for the test preparation of composition 25 was used, except that polysorbate 80 was excluded, to prepare composition 26 (a suspension). In addition, uncrushed sirolimus, polysorbate 80, and purified water were mixed. Subsequently, wet pulverization using a bead mill was performed over about 1 min. Then, hypromellose TC5 (registered trademark), sodium citrate hydrate, sodium edetate hydrate, and sodium chloride were mixed. After that, a pH adjuster (hydrochloric acid and/or sodium hydroxide) and purified water were added thereto to have a total volume of 100 mL. In this way, a test preparation of composition 27 (a suspension) was prepared. The concentration of each component contained in each test preparation was as shown in Table 9.

[Table 9]

| Test Preparation [% (w/v)] | Composition 25 | Composition 26 | Composition 27 |
|---|---|---|---|
| Sirolimus | 0.1 | 0.1 | 0.1 |
| Polysorbate 80 | 0.1 | - | 0.1 |
| Hypromellose | 0.001 | 0.001 | 0.001 |
| Sodium edetate hydrate | 0.01 | 0.01 | 0.01 |
| Sodium citrate hydrate | q.s. | | |
| Sodium chloride | q.s. | | |
| pH adjuster | q.s. | | |
| Purified water | q.s. | | |
| pH | 7.0 | 7.0 | 5.0 |

(2) Test Method

Here, 5 mL of each of the test preparations of compositions 25 to 27 was filled into a sterile container, and tightly sealed. Each container was stored for 2 weeks at a temperature of 40°C and a humidity of 20%. At the time of immediately after filling and after 2 weeks of storage, the same method as in the section "2. Agglomeration Evaluation and Stability Test" was used to measure the average particle size and calculate the particle size increase rate.

(3) Test Results and Discussion

Table 10 shows the test results.

[Table 10]

| Test Results | Storage Conditions (Temperature, Humidity) | Composition 25 | Composition 26 | Composition 27 |
|---|---|---|---|---|
| Average particle size (μm) | Immediately after filling | 63.2 | 82.2 | 45.2 |
| | 40°C, 20% | 183 | 279 | 101 |
| Particle size increase rate | 40°C, 20% | 2.90 | 3.39 | 2.23 |

Table 10 demonstrates that in the test preparations containing uncrushed sirolimus or the test preparation obtained by crushing for a short period of time, the average particle size is large immediately after filling or later, further agglomeration is likely to occur during storage. Thus, it is indicated that as the average particle size of sirolimus becomes larger, agglomeration is more likely to occur.

5. Agglomeration Test

(1) To Prepare Test Preparation

The same method as for the test preparation of composition 5 in the section "2. Agglomeration Evaluation and Stability Test" was used to prepare each test preparation (each was a suspension) of composition 28 or 29. The concentration of each component contained in each test preparation was as shown in Table 11.

[Table 11]

| Test Preparation [% (w/v)] | Composition 28 | Composition 29 |
|---|---|---|
| Sirolimus | 0.01 | 0.01 |
| Polysorbate 80 | 0.01 | 0.001 |
| Hypromellose | 0.001 | 0.001 |
| Sodium edetate hydrate | 0.01 | 0.01 |
| Sodium citrate hydrate | q.s. | |
| Sodium chloride | q.s. | |
| pH adjuster | q.s. | |
| Purified water | q.s. | |
| pH | 5.0 | |

(2) Test Method

Here, 5 mL of each of the test preparations of compositions 28 to 29 was filled into a sterile container, and tightly sealed. Each container was stored for 2 weeks at a temperature of 40°C and a humidity of 20%. At the time of immediately after filling and after 2 weeks of storage, the same method as in the section "2. Agglomeration Evaluation and Stability Test" was used to measure the average particle size and calculate the particle size increase rate.

(3) Test Results and Discussion

Table 12 shows the test results.

[Table 12]

| Test Results | Storage Conditions (Temperature, Humidity) | Composition 28 | Composition 29 |
|---|---|---|---|
| Average particle size (μm) | Immediately after filling | 0.47 | 0.31 |
| | 40°C, 20% | 0.61 | 0.46 |
| Particle size increase rate | 40°C, 20% | 1.30 | 1.48 |

As shown in Table 12, in the case where the content of surfactant based on 1 part by weight of sirolimus was 1 part by weight (in the case of composition 28) or in the case where the content of surfactant based on 1 part by weight of sirolimus was 0.1 parts by weight (in the case of composition 29), agglomeration was found to be unlikely to occur even when the concentration of the surfactant in the test preparation was low.

6. Agglomeration Test

(1) To Prepare Test Preparation

The same method as for the test preparation of composition 5 in the section "2. Agglomeration Evaluation and Stability Test" was used to prepare each test preparation (any of them was a suspension) of compositions 30 to 35 containing respective components designated in Table 13. The concentration of each component contained in each test preparation was as shown in Table 13.

[Table 13]

| Test Preparation [% (w/v)] | Composition 30 | Composition 31 | Composition 32 | Composition 33 | Composition 34 | Composition 35 |
|---|---|---|---|---|---|---|
| Sirolimus | 0.1 | 0.1 | 0.1 | 0.1 | 0.1 | 0.1 |
| Polysorbate 80 | 0.1 | - | 0.1 | 0.1 | 0.1 | 0.1 |
| CO-35 | - | 0.1 | - | - | - | - |
| Hypromellose | 0.01 | 0.01 | - | - | - | - |
| CMC-sodium | - | - | 0.01 | - | - | - |
| HA | - | - | - | 0.01 | - | - |
| PVA | - | - | - | - | 0.01 | - |
| PVP | - | - | - | - | - | 0.01 |
| Sodium edetate hydrate | 0.01 | 0.01 | 0.01 | 0.01 | 0.01 | 0.01 |
| Concentrated glycerin | q.s. | | | | | |
| Sodium dihydrogen phosphate hydrate | q.s. | | | | | |
| pH adjuster | q.s. | | | | | |
| Purified water | q.s. | | | | | |
| pH | 5.0 | | | | | |

(2) Test Method

Here, 5 mL of each of the test preparations of compositions 30 to 35 was filled into a sterile container, and tightly sealed. The compositions 30 and 31 were stored for 19 months at room temperature and the compositions 32 to 35 were stored for 28 months at room temperature. At the time of immediately after filling and after storage, the same method as in the section "2. Agglomeration Evaluation and Stability Test" was used to measure the average particle size and calculate the particle size increase rate.

(3) Test Results and Discussion

Table 14 shows the test results.

[Table 14]

| Test Results | Storage Conditions | Compo-sition 30 | Composition 31 | Composition 32 | Composition 33 | Composition 34 | Composition 35 |
|---|---|---|---|---|---|---|---|
| Average particle size (μm) | Immediately after filling | 0.62 | 0.73 | 0.55 | 0.63 | 0.53 | 0.53 |
| | Storage at room temperature | 0.82 | 0.79 | 0.61 | 0.69 | 0.61 | 0.56 |
| Particle size increase rate | Storage at room temperature | 1.32 | 1.08 | 1.11 | 1.10 | 1.15 | 1.06 |

Table 14 has demonstrated that the type of surfactant or the type of dispersant do not largely affect the particle aggregation.

INDUSTRIAL APPLICABILITY

The present invention provides an aqueous suspension composition containing poorly water-soluble sirolimus for ophthalmology, in particular, used for topical administration, such as a less invasive eye drop.

## Claims

1. An ophthalmic aqueous suspension composition comprising sirolimus or a salt thereof and a surfactant, wherein the aqueous suspension composition has a pH of 4 to 6.

2. The aqueous suspension composition according to claim 1, wherein a content ratio of the surfactant to sirolimus or a salt thereof is more than 0.01 parts by weight based on 1 part by weight of a content of sirolimus or a salt thereof, and sirolimus or a salt thereof in the aqueous suspension composition has an average particle size of 45 μm or less.

3. The aqueous suspension composition according to claim 1, wherein a content ratio of the surfactant to sirolimus or a salt thereof is more than 0.01 parts by weight based on 1 part by weight of a content of sirolimus or a salt thereof, and sirolimus or a salt thereof in the aqueous suspension composition has an average particle size of 15 μm or less.

4. The aqueous suspension composition according to claim 1, wherein a content ratio of the surfactant to sirolimus or a salt thereof is more than 0.01 parts by weight based on 1 part by weight of a content of sirolimus or a salt thereof, and sirolimus or a salt thereof in the aqueous suspension composition has an average particle size of 10 μm or less.

5. The aqueous suspension composition according to any one of claims 1 to 4, wherein the aqueous suspension composition has a pH of 5.

6. The aqueous suspension composition according to any one of claims 1 to 5, wherein a content of sirolimus or a salt thereof is from 0.01 to 1% (w/v).

7. The aqueous suspension composition according to any one of claims 1 to 6, wherein sirolimus or a salt thereof has an average particle size of 2.5 μm or less.

8. The aqueous suspension composition according to any one of claims 1 to 7, wherein the surfactant is one or more selected from the group consisting of polyoxyethylene fatty acid esters, polyoxyethylene sorbitan fatty acid esters, polyoxyethylene hydrogenated castor oils, polyoxyl castor oils, and polyoxyethylene alkyl ether phosphates.

9. The aqueous suspension composition according to any one of claims 1 to 7, wherein the surfactant is one or more selected from the group consisting of polyoxyl 40 stearate, polysorbate 80, polyoxyl 35 castor oil, and sodium polyoxyethylene cetyl ether phosphate.

10. The aqueous suspension composition according to any one of claims 1 to 7, wherein the surfactant is polysorbate 80.

11. The aqueous suspension composition according to any one of claims 1 to 10, comprising a dispersant.

12. The aqueous suspension composition according to claim 11, wherein the dispersant is one or more selected from the group consisting of cellulosic polymers, polyalcohols, polyvinylpyrrolidone, and mucopolysaccharides.

13. The aqueous suspension composition according to any one of claims 1 to 12, further comprising one or more selected from the group consisting of buffering agents, tonicity agents, stabilizers, antioxidants, preservatives, and pH adjusters.

14. The aqueous suspension composition according to claims 13, wherein the preservatives are one or more selected from the group consisting of invert soaps, parabens, sorbic acid or salts thereof, chlorobutanol, and silver nitrate.

15. The aqueous suspension composition according to any one of claims 1 to 14, which is an eye drop.

**16.** An ophthalmic aqueous suspension composition comprising sirolimus or a salt thereof and polysorbate 80, wherein

a content of sirolimus or a salt thereof is from 0.01 to 1% (w/v),
a content ratio of the polysorbate 80 to sirolimus or a salt thereof is from 0.1 to 10 parts by weight based on 1 part by weight of a content of sirolimus or a salt thereof,
sirolimus or a salt thereof in the aqueous suspension composition has an average particle size of 2.5 μm or less, and
the aqueous suspension composition has a pH of 4 to 6.

**17.** A method of suppressing agglomeration of sirolimus or a salt thereof in an aqueous suspension composition comprising sirolimus or a salt thereof and a surfactant, comprising adjusting a pH of the aqueous suspension composition to 4 to 6 and setting an average particle size of sirolimus or a salt thereof in the aqueous suspension composition to 45 μm or less.

**18.** A method of suppressing agglomeration of sirolimus or a salt thereof in an aqueous suspension composition comprising sirolimus or a salt thereof and a surfactant, comprising adjusting a pH of the aqueous suspension composition to 4 to 6 and setting an average particle size of sirolimus or a salt thereof in the aqueous suspension composition to 15 μm or less.

**19.** A method of suppressing agglomeration of sirolimus or a salt thereof in an aqueous suspension composition comprising sirolimus or a salt thereof and a surfactant, comprising adjusting a pH of the aqueous suspension composition to 4 to 6 and setting an average particle size of sirolimus or a salt thereof in the aqueous suspension composition to 10 μm or less.

**20.** A method of suppressing decomposition of sirolimus in an aqueous suspension composition comprising sirolimus or a salt thereof and a surfactant, comprising adjusting a pH of the aqueous suspension composition to 4 to 6.

**INTERNATIONAL SEARCH REPORT**

International application No. PCT/JP2020/048738

A. CLASSIFICATION OF SUBJECT MATTER

A61K 31/436(2006.01)i; A61K 9/10(2006.01)i; A61K 47/26(2006.01)i; A61K 47/38(2006.01)i; A61P 27/02(2006.01)i
FI: A61K31/436; A61K9/10; A61K47/26; A61K47/38; A61P27/02

According to International Patent Classification (IPC) or to both national classification and IPC

B. FIELDS SEARCHED

Minimum documentation searched (classification system followed by classification symbols)
A61K31/436; A61K9/10; A61K47/26; A61K47/38; A61P27/02

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X<br>A | CN 106420607 A (BEIJING NUOKANGDA PHARMACEUTICAL TECHNOLOGY CO., LTD.) 22 February 2017 (2017-02-22) claim 1 | 1-4, 6-20<br>5 |
| X<br>A | CN 107334734 A (ZHEJIANG UNIVERSITY) 10 November 2017 (2017-11-10) claim 6, paragraphs [0012], [0027] | 1-4, 6-20<br>5 |
| X<br>A | JP 2010-536797 A (MACUSIGHT, INC.) 02 December 2010 (2010-12-02) claim 7, table 1, formulations 12, 14, 41, 42 | 1-4, 6-20<br>5 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 February 2021 (02.02.2021) | 16 February 2021 (16.02.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/048738

| Patent Documents referred in the Report | Publication Date | Patent Family | Publication Date |
|---|---|---|---|
| CN 106420607 A | 22 Feb. 2017 | (Family: none) | |
| CN 107334734 A | 10 Nov. 2017 | (Family: none) | |
| JP 2010-536797 A | 02 Dec. 2010 | US 2009/0074786 A1<br>claim 7, table 1,<br>Form. 12, 14, 41, 42<br>WO 2009/023877 A2<br>EP 2187743 A2<br>KR 10-2010-0055482 A<br>CN 101827523 A<br>ES 2507078 T | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H5194212 A
- JP 2010540682 A
- WO 2014142146 A1


### Non-patent literature cited in the description


- General Rules for Preparations of the Japanese Pharmacopoeia